# EUROPEAN PATENT SPECIFICATION

(11) **EP 4 405 090 B1**
(45) Date of publication and mention of the grant of the patent: **27.05.2026**
(21) Application number: 22777650.7
(22) Date of filing: 22.09.2022
(51) Int. Cl.: B01F 27/191, B01F 27/2121, B01F 27/2124, B01F 27/07, B01F 27/84, G01N 13/00, G01N 33/15

(54) **STIRRING DEVICE FOR STIRRING A BIPHASIC LIQUID**
RÜHRVORRICHTUNG ZUM RÜHREN EINER ZWEIPHASIGEN FLÜSSIGKEIT
DISPOSITIF D'AGITATION POUR AGITER UN LIQUIDE BIPHASIQUE

(30) Priority: 24.09.2021 EP 21198808
(43) Date of publication of application: 31.07.2024
(73) Proprietor: Merck Patent GmbH, 64293 Darmstadt (DE)
(72) Inventor: JEDE, Christian, 64293 DARMSTADT (DE); MEINERS, Kirstin, 64293 DARMSTADT (DE); WEDEL, Marcel, 64293 DARMSTADT (DE); BOGDAHN, Malte, 64293 Darmstadt (DE)
(74) Representative: Merck Patent Association
(86) International application number: PCT/EP2022/076310
(87) International publication number: WO 2023/046807

(56) References cited:
- CN-B- 105 597 591
- DE-U1- 202014 005 974
- GB-A- 2 503 690
- JP-U- H0 192 235
- KR-A- 20190 046 095

## Description

### Technical Field

The present invention relates to a stirring device for stirring a biphasic liquid with a stirring shaft that can be mounted with a mounting end into a stirring drive, with a first paddle that is arranged in a rotationally fixed manner at or near a stirring end of the stirring shaft that is opposite to the mounting end, and with a second paddle that is arranged in a rotationally fixed manner at the stirring shaft in between the mounting end and the first paddle.

### State of the Art

The prediction of in vivo dissolution, precipitation and absorption of poorly soluble drugs represents a major task in today's drug discovery and development. Especially during gastrointestinal transit, in vitro assays often overpredict pH-dependent drug precipitation due to a missing absorption step. In vivo, drug precipitation is most likely less pronounced since high local drug concentrations are decreased by continuous drug removal, i.e. drug absorption. Thus, reliable in vitro studies are required to accurately predict in vivo behavior of a drug.

The gastrointestinal transit of a drug can be mimicked using in vitro transfer systems. They usually comprise two dissolution vessels, one simulating the stomach and the other one simulating the intestine, connected by a peristaltic pump. To account for simulated drug absorption during dissolution testing, an organic phase, e.g. octanol or decanol, can be added on top of an aqueous dissolution medium.

Due to the different characteristics of the aqueous phase and the organic phase, both liquids tend to separate from each other and form a biphasic system with the organic phase on top of the aqueous dissolution medium. Drug absorption is simulated by the partitioning of dissolved drug from the aqueous phase of the aqueous dissolution medium into the organic phase. In such a model and transfer system, the aqueous dissolution medium volume increases during gastrointestinal transit, as simulated gastric contents are continuously being transferred from a first dissolution vessel that represents the gastric compartment into a second dissolution vessel representing the simulated intestinal compartment.

During such a dissolution testing, constant stirring of both the aqueous phase and the organic phase within the dissolution vessel is required in order to provide for a homogenous distribution of the dissolved drug in both phases. Due to the separation of the organic phase from the aqueous dissolution medium and the resulting phase boundary, a stirring motion solely created within the aqueous dissolution medium or within the organic phase will not affect the other phase in a similar manner. Thus, a stirring device that is used for a biphasic liquid during such a drug absorption test usually comprises two paddles mounted at a common stirring shaft at an axial distance towards each other. A first paddle is mounted at a stirring end of the stirring shaft, and a second paddle is mounted at a distance to the first paddle in between the first paddle and the opposite end of the stirring shaft. During execution of a dissolution test, the stirring shaft is mounted to a rotary drive of a stirring system and projects into the biphasic liquid. A rotational movement caused by the rotary drive results in a rotating stirring movement of the first and second paddle and a corresponding stirring movement of the aqueous dissolution medium and of the organic phase. The filling level of the aqueous dissolution medium within the dissolution vessel and the filling level of the organic phase on top of the aqueous dissolution medium are adapted to the distance between the first and second paddle such that at the beginning of the drug absorption test the first paddle is arranged within the aqueous dissolution medium and stirs the aqueous dissolution medium, whereas the second paddle is arranged within the organic phase and stirs the organic phase. The phase boundary runs between the first and second paddle.

However, during the execution of the drug absorption test additional dissolution liquid is pumped into the dissolution vessel. The increase of the volume of the aqueous dissolution medium results in a corresponding rise of the phase boundary between the increasing volume of the aqueous dissolution medium and the organic phase on top of the aqueous dissolution medium. After some time, the phase boundary may rise to the point where it reaches the second paddle. Then, the rotating second paddle creates unwanted turbulences within the phase boundary, which adversely affects the test results and significantly reduces the informative value and the comparability of the drug absorption test. Thus, the duration of such a dissolution test is limited by the distance between the first and second paddle. However, a large distance results in incomplete stirring of the two phases near the phase boundary, which also affects the test results.

Thus, there is a need to provide for a stirring device that allows for a smooth and controlled stirring motion within both phases, namely within the aqueous dissolution medium and within the organic phase, during a long duration of the dissolution test and a continuously increasing volume of the aqueous dissolution medium during the execution of the dissolution test that also simulates the transfer of additional contents into the biphasic liquid by adding of additional aqueous dissolution medium into the biphasic liquid.

Document DE 20 2014 005974 U1 discloses a stirring device in accordance with the preamble of claim 1.

### Summary of the Invention

The present invention discloses a stirring device as described above, whereby the second paddle is mounted with respect to the stirring shaft in such a manner that it can be displaced in an axial direction along the stirring shaft, which allows for a change of an axial distance between the second paddle and the mounting end of the stirring shaft. Thus, the axial displacement of the second paddle with respect to the stirring shaft allows to compensate for the rising filling level of the aqueous dissolution medium within the vessel without need for any axial displacement of the stirring shaft. The axial position of the second paddle can be preset and continuously adjusted to staying within the organic phase. Furthermore, the position of the second paddle can be continuously adjusted to be at a constant distance above the phase boundary. It is also possible and will be described below that the position of the second paddle automatically follows any rise of an upper filling level of the organic phase, e.g. by making use of a floating device that is connected to the second paddle. Then, the stirring motion that is created within the organic phase remains essentially the same during the dissolution test, even though the phase boundary continually rises during the dissolution test. It is understood that the first paddle or the second paddle or both paddles may each comprise one or more plates or paddle elements that extend radially outwards on one side or in several radial directions of the stirring shaft.

It is possible to automatically monitor the filling level of either the biphasic liquid within the vessel or the filling level of the aqueous dissolution medium, i.e. the phase boundary between the aqueous dissolution medium and the organic phase. The axial position of the second paddle can be continually adjusted or adjusted at intervals to remain at a constant distance to either the upper filling level of the organic phase or to the phase boundary and the filling level of the aqueous dissolution medium, depending on which filling level is monitored. Such a monitoring of a filling level can be performed e.g. by optical measurements or by using a buoyancy body that floats on top of the organic phase.

The mounting of the first paddle can be such that the first paddle rises together with the second paddle along the stirring shaft, or that the first paddle remains at a fixed position at the stirring end of the stirring shaft. It is also possible that the first paddle is also displaced along the axial direction towards the mounting end of the stirring shaft, but at a smaller displacement rate, resulting in an increasing distance between the first and second paddle. The displacement rate can be e.g. half of the rising rate of the phase boundary that equals the filling level of the aqueous dissolution medium, resulting the first paddle to remain in the same relative height within the volume of the aqueous dissolution medium.

According to an embodiment of the invention, the second paddle is mounted at a hollow shaft which surrounds the stirring shaft and is axially displaceable relative to the stirring shaft. As the first paddle and the second paddle are mounted on different shafts, namely the stirring shaft and the surrounding hollow shaft, both paddles can rotate differently, e.g. at a different rotational speed or in an opposite rotational direction. This requires a suitable rotary drive mechanism and the possibility to mount the stirring shaft as well as the surrounding hollow shaft to the rotary drive mechanism. However, different rotational movement of the first and second paddle allows for a large variety of testing parameters and simulations. Furthermore, the axial position of the first paddle and of the second paddle with respect to the mounting end of the stirring shaft can be easily preset at the most effective position for causing a homogenous and constant stirring motion within the aqueous dissolution medium and within the organic phase, and each of the axial positions of the first and second paddle can be varied differently and independently of each other.

According to another embodiment of the invention that allows for a cost-effective manufacture and operation of the stirring device, the second paddle is mounted in a rotationally fixed manner at the stirring shaft. Thus, both the first and second paddle are mounted at the stirring shaft. Such an embodiment does not require an additional hollow shaft and a suitably designed rotary drive.

Due to the rotationally fixed mounting of the first and second paddle to the same stirring shaft, both the first and second paddle perform an identical rotational movement during the execution of the dissolution test. However, for many applications including standard dissolution testing methods the same rotational movement of the first paddle and of the second paddle are considered advantageous or mandatory.

In order to provide for a constant axial distance between the first and second paddle along the stirring shaft, the first paddle is rigidly connected to the second paddle. As the second paddle is mounted axially displaceable to the stirring shaft, also the first paddle must be mounted axially displaceable with respect to the stirring shaft. An axial displacement of the second paddle then causes a similar axial displacement of the first paddle, which will move away from the stirring end as the second paddle rises during the execution of the dissolution test. A rigid connection between the first and second paddle can be provided by e.g. manufacturing the first and second paddle in one piece. The two paddles can be connected e.g. by a hollow shaft that extends from the first to the second paddle. It is also possible to attach the first and second paddle to one or more rigid connecting elements that run parallel to the stirring shaft.

According to a favorable aspect of the invention, the first paddle is rigidly connected to the stirring end of the shaft. Thus, the first paddle can be manufactured in one piece with the stirring shaft. It is also possible to separately manufacture the first paddle and the stirring shaft, and to attach the first paddle at the stirring end of the stirring shaft. The fixed position of the first paddle with respect to the stirring shaft allows for a cost-effective manufacture of the stirring device. The number and the proportion of movable components of the stirring device can be reduced to a minimum, which helps to provide for a reliable operation of the stirring device over a long period of use.

It is considered a very advantageous embodiment of the invention that the stirring shaft can have a non-rotationally symmetric shape, and that the second paddle has a continuous recess with a cross-sectional area that is adapted to the non-rotationally symmetric shape of the stirring shaft such that the second paddle is slidable in the axial direction along the shaft, but mounted in a rotationally fixed manner at the stirring shaft. The stirring shaft can have e.g. a quadratic or a rectangular cross-sectional area, or any other polygonal or non-rotationally symmetrical shape of the cross-sectional area. Preferably, the shape of the cross-sectional area of the stirring shaft does not change within a displacement section along the axial direction of the stirring shaft, whereby the displacement section presets the range of maximum displacement of the second paddle along the stirring shaft in the axial direction. For many applications, the shape of the cross-sectional area of the stirring shaft does not vary at all or only varies at the mounting end or at the stirring end or at both ends, e.g. to provide for a secure fastening of the stirring shaft with the rotary drive or with the first paddle.

The second paddle can be slidably arranged at the stirring shaft that runs through the continuous recess. Due to the non-rotationally symmetric shape of the stirring shaft and the suitably adapted continuous recess within the second paddle, the second paddle follows any rotational movement of the stirring shaft without need for any further mechanism of action or operative connection.

According to one embodiment of the invention, the second paddle can be displaced in the axial direction with a drive device. The drive device can comprise a motor that acts on a hoisting rope or a tooth rack that is connected to the second paddle and that can be used to rise or displace the second paddle along the stirring shaft. The drive device can also comprise a pneumatic or hydraulic drive mechanism with a fluidic connection that acts on the second paddle and lifts or lowers the second paddle along the stirring shaft. Such a drive device allows for a controlled displacement of the second paddle along the stirring shaft. In combination with a control device that monitors the position of the phase boundary within the vessel, at any time during a dissolution test the second paddle can be displaced to a position within the organic phase with a predefined distance to the phase boundary as well as to the upper filling level of the organic phase. It is also possible to predefine different positions of the second paddle, i.e. different distances to the phase boundary for different dissolution tests, or to vary the position during a single dissolution test.

In yet another embodiment that is considered very advantageous for a cost-effective manufacture and reliable operation of the stirring device, the second paddle is connected to a buoyancy body that floats on an upper surface of the biphasic fluid, resulting in a rise of the second paddle as the buoyancy body rises with a rising upper surface of the biphasic fluid. Such a buoyancy body can have any shape and volume, provided that the buoyancy force that acts on the buoyancy body is large enough to carry along the second paddle that is connected to the buoyancy body. The distance between the second paddle and the upper surface of the biphasic fluid can be preset by the design of the buoyancy body and the connection of the second paddle with it. As the volume of the organic phase usually remains constant during a dissolution test, presetting a distance between the second paddle and the upper filling level of the organic phase also presets the distance of the second paddle to the phase boundary between the aqueous dissolution medium and the organic phase on top of the aqueous dissolution medium. If the volume of the aqueous dissolution medium increases during the execution of a dissolution test, the phase boundary rises, and the volume as well as the upper filling level of the organic phase rises in a similar manner, provided that the cross-sectional shape of the vessel remains constant along the vertical direction. Thus, by using a buoyancy body and connecting the second paddle to the buoyancy body the position of the second paddle remains constant with respect to the upper filling level as well as to the phase boundary, resulting in a very constant and homogeneous stirring motion of the organic phase even though the position of the organic phase changes with the increase of the aqueous dissolution medium.

According to an embodiment of the invention the second paddle can be made to be the buoyancy body or to comprise the buoyancy body e.g. as upper part of the second paddle. However, in order to not disturb the stirring motion of the organic phase by adding the buoyancy body to the second paddle, the buoyancy body is a rotational body. Even though the buoyancy body can be rigidly connected to the second paddle and then follows any rotational movement of the second paddle, due to shape of the rotational body of the buoyancy body, there will be only a very small amount of additional stirring motion or turbulences transmitted to the organic phase by the buoyancy body.

Preferably, the buoyancy body has a conical shape with a larger diameter facing the mounting end of the stirring shaft. Such a shape further reduces any unwanted transfer of stirring motion or turbulences caused by the buoyancy body into the organic phase of the biphasic liquid.

According to another embodiment of the invention, the buoyancy body has a discoidal shape. A discoidal shape with a flat lower surface that floats on the organic phase has a different contact area than a conical shape and does not penetrate deeply below the upper filling level of the organic phase. Furthermore, the radial dimension of the discoidal shape can be adapted to the diameter of the dissolution vessel in order to cover a large surface area of the biphasic liquid, whereby the escape of volatile components like e.g. carbon dioxide or other gases that are created or accumulated during the dissolution test can be reduced or largely prevented.

In order to decouple a rotational movement of the second paddle from the buoyancy body, it is considered an advantageous option of the invention that the buoyancy body is connected to the second paddle via a thrust bearing. Thus, the buoyancy body can remain stationary, even though the second paddle rotates and generates a stirring motion within the organic phase of the biphasic liquid. The thrust bearing may comprise a ball bearing or a rolling bearing. Preferably the thrust bearing is arranged above the upper filling level of the biphasic liquid in order to avoid any contact of the organic phase with the thrust bearing during the performance of the dissolution test, which might cause unwanted turbulences within the organic phase.

According to the invention, the stirring device comprises an adapter shaft that surrounds the stirring shaft and that can be mounted in a rotationally fixed manner on top of the first paddle, whereby an outer surface of the adapter shaft has a non-rotationally symmetric shape that allows for mounting the second paddle with respect to the adapter shaft in such a manner that it can be displaced in an axial direction along the adapter shaft and correspondingly in an axial direction along the stirring shaft that is surrounded by the adapter shaft. By making use of an adapter shaft that can be slid over the stirring shaft and allows for a rotationally fixed mounting of the second paddle at the adapter shaft, conventional stirring devices with a first paddle that is arranged at the end of a stirring shaft with a circular cross-sectional area can be used and modified into a stirring device according to this invention. Thus, the adapter shaft and the second paddle as well as e.g. a buoyancy body attached thereto can be offered as an aftermarket kit that allows for upgrading already existing conventional stirring devices with only one paddle into stirring devices according to this invention, which provide for the advantages of a stirring device with two paddles that each remain within the respective phase of a biphasic liquid. Furthermore, due to the continuous use of the first paddle of the conventional stirring device, dissolution test results that have been performed with such a conventional stirring device can be matched with and compared to measurement results that have been obtained by using the upgraded stirring device according to this invention.

It is understood that the stirring device described above can be used for stirring any biphasic liquid and is not limited to stirring a aqueous dissolution medium and an organic phase during the execution of a dissolution test for drugs. Furthermore, it is also possible to use the stirring device described above for stirring a homogeneous liquid within a vessel with or without a rising upper filling level during the operation of the stirring device.

### Brief description of the drawings

The present invention will be more fully understood, and further features will become apparent, when reference is made to the following detailed description and the accompanying drawings. The drawings are merely representative and are not intended to limit the scope of the claims. In fact, those of ordinary skill in the art may appreciate upon reading the following specification and viewing the present drawings that various modifications and variations can be made thereto without deviating from the innovative concepts of the invention. Like parts depicted in the drawings are referred to by the same reference numerals.
Figure 1 illustrates a schematic representation of an experimental setup for performing a dissolution test with two dissolution vessels and a pump device that continuously transfers a small amount of a first aqueous dissolution medium from a first dissolution vessel to a second dissolution vessel that holds a biphasic liquid with a second aqueous dissolution medium and an organic phase,
Figure 2 illustrates the experimental setup shown in Figure 1 at a later time, i.e. after a large amount of the first dissolution medium has been transferred from the first dissolution vessel into the second dissolution vessel,
Figure 3 illustrates a schematic representation of the second dissolution vessel with the biphasic liquid and with a stirring device, whereby the stirring device comprises a first paddle and a second paddle that are used for stirring both phases of the biphasic liquid,
Figure 4 illustrates the second dissolution vessel with the biphasic liquid and the stirring device shown in Figure 1 after the volume of the lower phase of the biphasic liquid has increased, whereby the second paddle has been displaced to follow the rise of the upper filling level and the phase boundary of the biphasic liquid,
Figure 5 illustrates the second dissolution vessel with a different embodiment of the stirring device after the volume of the lower phase of the biphasic liquid has increased, whereby the second paddle and the first paddle are both displaced to follow the rise of the upper filling level and the phase boundary of the biphasic liquid,
Figure 6 illustrates a second dissolution vessel with a stirring device with a buoyancy body that is connected to the second paddle,
Figure 7 illustrates a perspective view of the stirring device shown in Figure 6,
Figure 8 illustrates a cross-sectional view of the stirring device shown in Figure 6,
Figure 9 illustrates a second dissolution vessel with yet another embodiment of the stirring device,
Figure 10 illustrates a side view of yet another embodiment of the stirring device, and
Figure 11 illustrates a cross-sectional view of the stirring device shown in Figure 10.

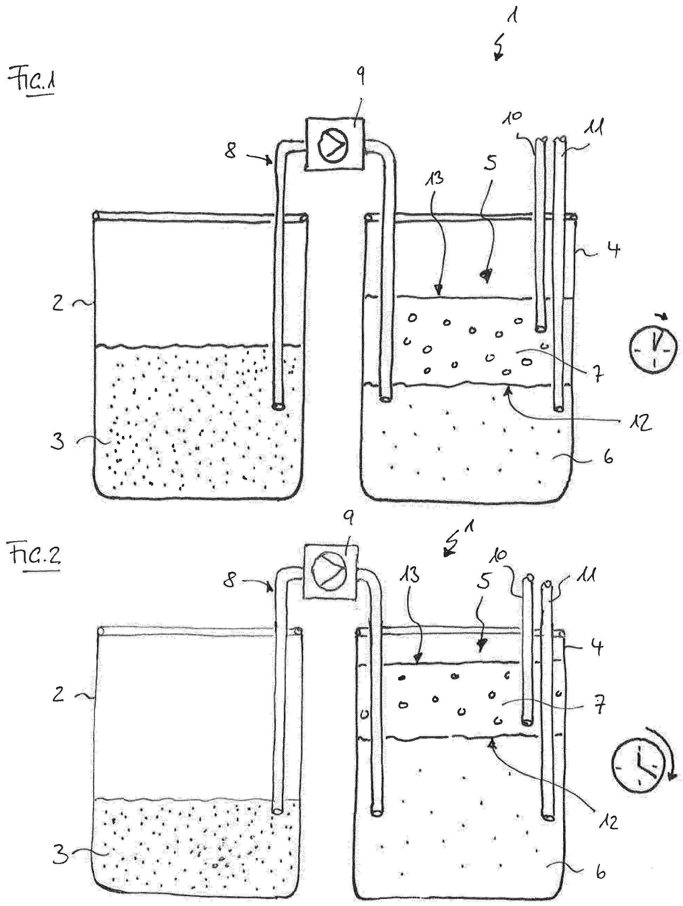

Figures 1 and 2 illustrate a schematic representation of an experimental setup 1 that is used for performing a dissolution test for simulating a drug dissolution within a gastric environment and within the intestinal environment. The experimental setup 1 comprises a first dissolution vessel 2 that is filled with a first aqueous dissolution medium 3 that represents the gastric environment. The first aqueous dissolution medium 3 has a physiologically relevant pH-value of approx. 1 to 5 and represents a stomach of a human body under different physiological conditions, i.e. prandial state or under co-administration with other drugs that affect a gastric pH-value. The experimental setup 1 further comprises a second dissolution vessel 4 that is filled with a biphasic liquid 5, whereby one phase of the biphasic liquid 5 is a second aqueous dissolution medium 6 and the other phase is an organic phase 7 like e.g. decanol or octanol. The pH-value of the second aqueous dissolution medium 6 is approx. 6.5 and represents an intestine of a human body. The two phases of the biphasic liquid 5 within the second dissolution vessel 4 tend to separate from each other, whereby the organic phase 7 accumulates above the second aqueous dissolution medium 6.

The experimental setup 1 further comprises a fluidic connection 8 between the first aqueous dissolution medium 3 within the first dissolution vessel 2 and the second aqueous dissolution medium 6 within the second dissolution vessel 4. A pumping device 9 that is arranged along the fluidic connection 8 allows for a transfer of a preset volume of the first aqueous dissolution medium 3 into the second dissolution vessel 4 and into the second aqueous dissolution medium 6 respectively. The pumping device 9 can be operated to perform a continuous transfer or to transfer small amounts at regular or irregular intervals. Two sampling conduits 10, 11 are connected to a sampling and analysis device that is not shown in the figures. By sampling and analyzing small probes of the biphasic liquid 5, the drug dissolution and partitioning within such a system can be monitored and investigated. The intestinal absorption of a drug is mainly simulated by the partitioning of the dissolved drug from the aqueous phase of the second aqueous dissolution medium 6 into the organic phase 7, i.e. the drug absorption into the organic phase 7. Comparable experimental setups have been described e.g. in Kostewicz, E. et al. 2004, Journal of Pharmacy and Pharmacology, Volume 56, Issue 1, January 2004, Pages 43-51; Jede, C. et al. 2019, International Journal of Pharmaceutics Volume 556, 10 February 2019, Pages 150-158; Xu, H. et al. 2018, European Journal of Pharmaceutical Sciences Volume 115, 30 March 2018, Pages 286-295.

Thus, at the beginning of a dissolution test, there is a large volume of the first aqueous dissolution medium 3 within the first dissolution vessel 2, and a similar or smaller volume of the second aqueous dissolution medium 6 within the second dissolution vessel 4, which is schematically illustrated in Figure 1. After some time during the execution of the dissolution test, the volume of the first aqueous dissolution medium 3 within the first dissolution vessel 2 has decreased, whereas the volume of the second aqueous dissolution medium 6 within the second dissolution vessel 4 has significantly increased, which is schematically illustrated in Figure 2. The increase of the volume of the second aqueous dissolution medium 6 causes a corresponding rise of a phase boundary 12 between the second aqueous dissolution medium 6 that accumulates at a lower region within the second dissolution vessel 4 and the organic phase 7 that accumulates at a higher region within the second dissolution vessel 4. The increase of the phase boundary 12 is matched by a corresponding increase of an upper filling level 13 of the organic phase 7 above the phase boundary 12.

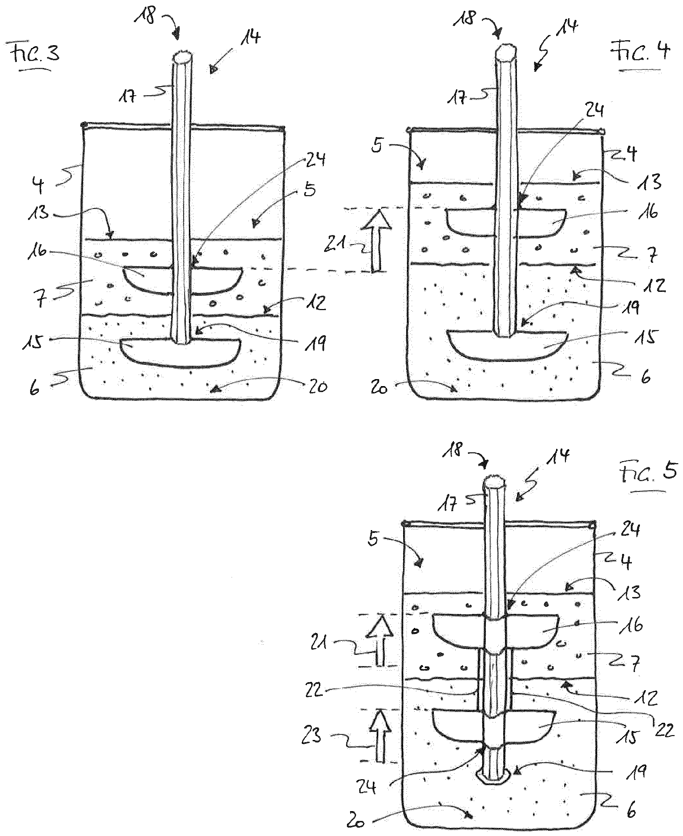

Figures 3 and 4 illustrate the second dissolution vessel 4 that is part of the experimental setup 1 for performing the dissolution test as described above. In order to provide for a homogeneous distribution of the dissolved drug within the second aqueous dissolution medium 6 as well as within the organic phase 7, both phases 6 and 7 of the biphasic liquid 5 must be stirred and kept in as uniform a stirring motion as possible. Thus, a stirring device 14 with a first paddle 15 and with a second paddle 16 projects into the second dissolution vessel 4. The stirring device 14 comprises a stirring shaft 17 that is mounted with a mounting end 18 to a rotary drive that is not shown in the figures. During execution of a dissolution test, operation of the rotary drive causes a rotating movement of the stirring shaft 17 as well as of the first paddle 15 and of the second paddle 16 that are mounted in a rotationally fixed manner to the stirring shaft 17. The rotating movement of the first and second paddle 15, 16 causes a stirring movement of the biphasic liquid 5.

The first paddle 15 is fixed at a stirring end 19 that is opposite to the mounting end 16 and projects into the second aqueous dissolution medium 6. Thus, the first paddle 15 is located near a bottom 20 of the second dissolution vessel 4.

Due to the different phases 6 and 7 of the biphasic liquid 5 and a phase boundary 12 that runs between the two phases 6 and 7, a stirring motion of the second aqueous dissolution medium 6 will not cause a similar stirring motion within the organic phase 7. Thus, the second paddle 16 is located within the volume of the organic phase 7 above the phase boundary 12 and below the upper filling level 13 of the organic phase 7, which is also the upper filling level 13 of the biphasic liquid 5 that is filled into the second dissolution vessel 4.

During the execution of dissolution testing method described above, the volume of the second aqueous dissolution medium 6 continually increases as additional quantities of first aqueous dissolution medium 3 with a preset proportion of the dissolved drug are introduced continually or at intervals into the second dissolution vessel 4. Thus, the phase boundary 12 as well as the upper filling level 13 will rise in accordance to the volume increase of the second aqueous dissolution medium 6. In order to keep the second paddle 16 at a distance to the phase boundary 12 as well as to the upper filling level 13, the second paddle 16 is displaced upwards in an axial direction along the stirring shaft 17. If the displacement of the second paddle 16 is coordinated with the rising of the phase boundary 12 or the similar rising of the upper filling level 13, the relative position of the second paddle 15 within the volume of the organic phase 7 remains constant. This allows for a very uniform and homogeneous stirring motion of the organic phase 7, resulting in meaningful and verifiable testing results. The upward displacement of the second paddle 16 is shown in Figure 4 and illustrated with an arrow 21.

Figure 5 schematically illustrates a different embodiment of the stirring device 14. Contrary to the embodiment shown in figures 3 and 4, the first paddle 15 is not fixed at the stirring end 19 of the stirring shaft 17, but also mounted slidably along the axial direction at the stirring shaft 17. Furthermore, the first paddle 15 is rigidly attached to the second paddle 16 by means of connecting rods 22. Thus, an axial displacement of the second paddle 16 causes an identical axial displacement of the first paddle 15. An upward displacement of the first and second paddle 15, 16 is illustrated with corresponding arrows 23 and 21. If the upward movement of the first and second paddle 15, 16 is coordinated with the upward movement of the phase boundary 12, the distance of both, the first paddle 15 and the second paddle 16 to the phase boundary 12 remains constant during the rise of the phase boundary 12 that is caused by an increase of volume of the second aqueous dissolution medium 6 during the execution of the dissolution test.

In both embodiments that are illustrated in Figures 3 to 5, the stirring shaft 17 has a polygonal cross-sectional area. The second paddle 16 has a continuous recess 24 with a polygonal cross-sectional area that is adapted to match the polygonal cross-sectional area of the stirring shaft 17 in such a manner that the second paddle 16 can slide along the axial direction of the stirring shaft 17, but is rotationally fixed with respect to the stirring shaft 17. Thus, the second paddle 16 can be displaced along the axial direction, but is forced to mimic all rotational movement of the stirring shaft 17. Within the embodiment that is illustrated in Figure 5, also the first paddle 15 has a continuous recess 24 that matches the stirring shaft 17 in order to provide for a rotationally fixed mounting of the first paddle 15 that can also slide along the axial direction and thus follow any axial displacement of the second paddle 16.

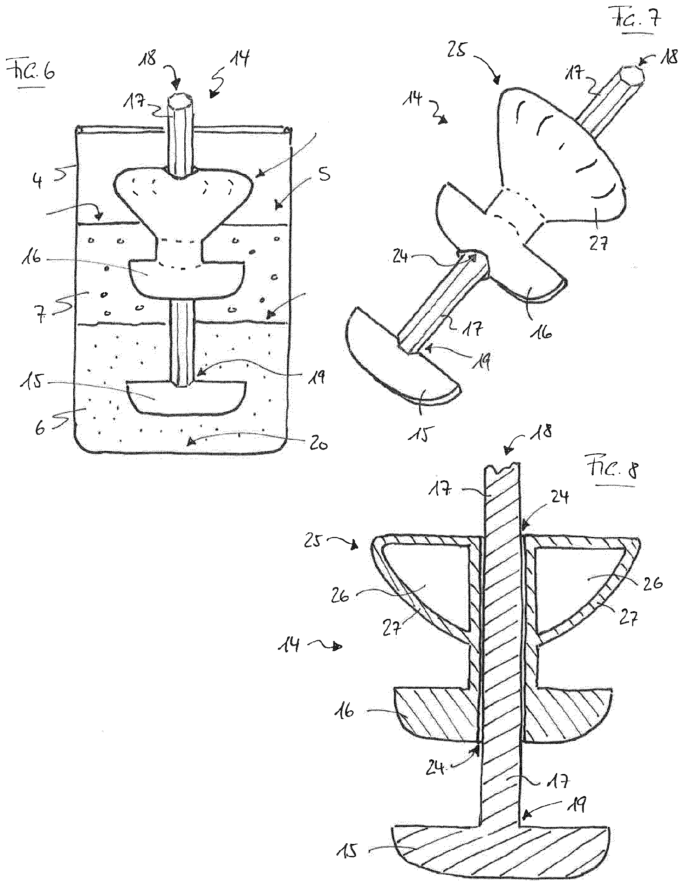

Figures 6 to 8 schematically illustrate an exemplary embodiment of the stirring device 14 with a buoyancy body 25. The buoyancy body 25 can be formed integrally with the second paddle 16, as illustrated in the Figures 6 to 8. It is also possible that the buoyancy body 25 is rigidly attached to the second paddle 16 by means of e.g. rods or ropes or a hollow shaft that extends upwards from the second paddle 16 up to the buoyancy body 25. It is also possible to connect the buoyancy body 25 to the second paddle 16 via a thrust bearing, which results in a decoupling of the rotational movement of the second paddle 16 from the floating movement of the buoyancy body 25.

The buoyancy body 25 has a hollow cavity 26 that is surrounded by a housing 27 that widens conically toward the top. The housing 27 has a rotationally symmetrical shape.

As shown in Figure 6, the buoyancy body 26 always floats on top of the organic phase 7, i.e. on the upper surface of the biphasic fluid 5 that equals the upper filling level 13 of the biphasic fluid 5. Thus, the buoyancy body 25 follows any rise of the upper filling level 13 of the biphasic liquid 5 within the second dissolution vessel 4 that is caused by the increase of volume of the aqueous dissolution medium 6 during execution of a dissolution test. The second paddle 16 that is connected to the buoyancy body 25 follows the displacement of the buoyancy body 25 and maintains a constant distance to the upper filling level 13 and also to the phase boundary 12 between the organic phase 7 and the second aqueous dissolution medium 6.

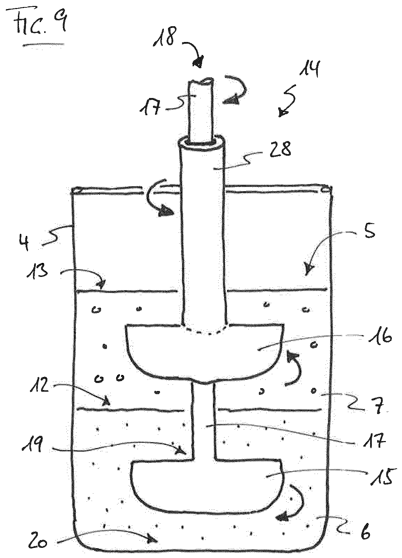

Figure 9 schematically illustrates yet another embodiment of the stirring device 14. The first paddle 15 is mounted in a rotationally fixed manner at the stirring end 19 of the stirring shaft 17. The cross-sectional area of the stirring shaft 17 is circular. The second paddle 16 is mounted at a hollow shaft 28 that surrounds the stirring shaft 17. Both, the stirring shaft 17 and the hollow shaft 28 are mounted to a rotary drive unit that forms a stirring drive. The rotary drive unit is capable to drive the stirring shaft 17 to a first rotational movement, and to drive the hollow shaft 287 to a second rotational movement that can be identical or different to the first rotational movement of the stirring shaft 17. Furthermore, the hollow shaft 28 can be axially displaced by the rotary drive unit or by a separate displacement unit that acts on the hollow shaft 28. Thus, the stirring system that is schematically illustrated in Figure 9 allows for individual adjustment of axial position and rotational movement of the first paddle 15 and of the second paddle 16.

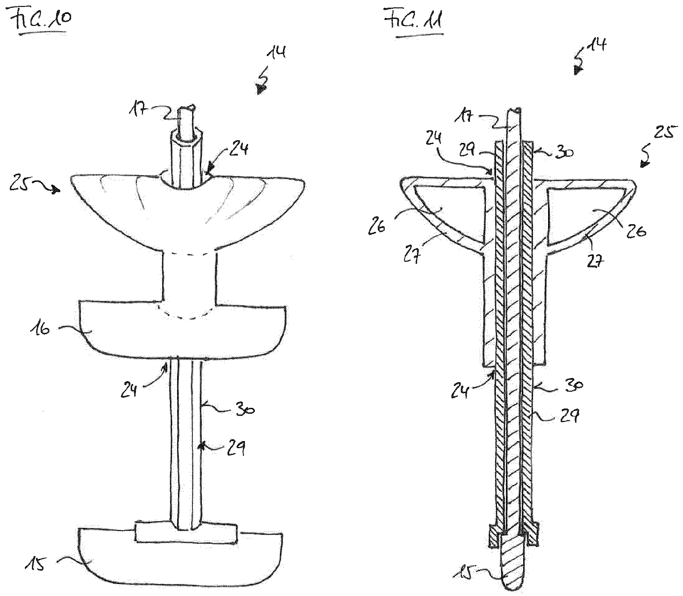

Figures 10 and 11 illustrate a side view and a cross-sectional view of yet another embodiment, in accordance with the invention, of the stirring device 14. An adapter shaft 29 is slid onto the stirring shaft 17 and surrounds the stirring shaft 17. The stirring shaft 17 has a circular cross-sectional area similar to many conventional stirring devices. The adapter shaft 29 is mounted on top of the first paddle 15 and provides for a positive locking with the first paddle 15, resulting in a rotationally fixed mounting of the adapter shaft 29 on the stirring shaft 17. An outer surface 30 of the adapter shaft 28 has a non-rotationally symmetric shape, e.g. a polygonal shape as illustrated within Figures 10 and 11. Thus, the second paddle 16 and the buoyancy body 25 can be mounted slidably along the axial direction, but in a rotationally fixed manner onto the adapter shaft 29.

## Claims

1. Stirring device (14) for stirring a biphasic fluid (5) with a stirring shaft (17) that can be mounted with a mounting end (18) into a stirring drive, with a first paddle (15) that is arranged in a rotationally fixed manner at or near a stirring end (19) of the stirring shaft (17) that is opposite to the mounting end (18) , and with a second paddle (16) that is arranged in a rotationally fixed manner at the stirring shaft (17) in between the mounting end (18) and the first paddle (15) , wherein the second paddle (16) is mounted with respect to the stirring shaft (17) in such a manner that it can be displaced in an axial direction along the stirring shaft (17) , which allows for a change of an axial distance between the second paddle (16) and the mounting end (18) of the stirring shaft (17), **characterized in that** the stirring device (14) comprises an adapter shaft (29) that surrounds the stirring shaft (17) and that can be mounted in a rotationally fixed manner on top of the first paddle (15) , whereby an outer surface (30) of the adapter shaft (29) has a non-rotationally symmetric shape that allows for mounting the second paddle (16) with respect to the adapter shaft (29) in such a manner that it can be displaced in an axial direction along the adapter shaft (29) and thus in an axial direction along the stirring shaft (17) that is surrounded by the adapter shaft (29).

2. Stirring device (14) according to claim 1, **characterized in that** the first paddle (15) is rigidly connected to the stirring end (19) of the stirring shaft (17).

3. Stirring device (14) according to one of the preceding claims, **characterized in that** the second paddle (16) is connected to a buoyancy body (25) that floats on an upper surface of the biphasic fluid (5) , resulting in a rise of the second paddle (16) as the buoyancy body (25) rises with a rising upper surface of the biphasic fluid (5).

4. Stirring device (14) according to claim 3, **characterized in that** the buoyancy body (25) is a rotational body.

5. Stirring device (14) according to claim 4, **characterized in that** the buoyancy body (25) has a conical shape with a larger diameter facing the mounting end (18) of the stirring shaft (17).

6. Stirring device (14) according to claim 4, **characterized in that** the buoyancy body (25) has a discoidal shape.

7. Stirring device (14) according to claims 4 to 6, **characterized in that** the buoyancy body (25) is connected to the second paddle (16) via a thrust bearing.

## Patentansprüche

1. Rührvorrichtung (14) zum Rühren eines zweiphasigen Fluids (5) mit einer Rührwelle (17),
die mit einem Montageende (18) in einen Rührantrieb einbaubar ist,
mit einem ersten Paddel (15), das drehfest an oder nahe einem dem Montageende (18) gegenüberliegenden Rührende (19) der Rührwelle (17) angeordnet ist,
und mit einem zweiten Paddel (16), das zwischen dem Montageende (18) und dem ersten Paddel (15) drehfest an der Rührwelle (17) angeordnet ist,
wobei das zweite Paddel (16) in Bezug auf die Rührwelle (17) so gelagert ist, dass es in axialer Richtung entlang der Rührwelle (17) verschiebbar ist, was eine Änderung des axialen Abstands zwischen dem zweiten Paddel (16) und dem Montageende (18) der Rührwelle (17) zulässt, **dadurch gekennzeichnet, dass**
die Rührvorrichtung (14) eine Adapterwelle (29) umfasst, die die Rührwelle (17) umgibt und die drehfest auf dem ersten Paddel (15) befestigt werden kann, wobei eine Außenfläche (30) der Adapterwelle (29) eine nicht-rotationssymmetrische Form aufweist, die es erlaubt, das zweite Paddel (16) in Bezug auf die Adapterwelle (29) so zu montieren, dass es in einer axialen Richtung entlang der Adapterwelle (29) und somit in einer axialen Richtung entlang der Rührwelle (17), die von der Adapterwelle (29) umgeben ist, verschoben werden kann.

2. Rührvorrichtung (14) nach Anspruch 1, **dadurch gekennzeichnet, dass** das erste Paddel (15) starr mit dem Rührende (19) der Rührwelle (17) verbunden ist.

3. Rührvorrichtung (14) nach einem der vorhergehenden Ansprüche, **dadurch gekennzeichnet, dass** das zweite Paddel (16) mit einem Auftriebskörper (25) verbunden ist, der auf einer oberen Oberfläche des zweiphasigen Fluids (5) schwimmt, was zu einem Ansteigen des zweiten Paddels (16) führt, wenn der Auftriebskörper (25) mit einer steigenden oberen Oberfläche des zweiphasigen Fluids (5) ansteigt.

4. Rührvorrichtung (14) nach Anspruch 3, **dadurch gekennzeichnet, dass** der Auftriebskörper (25) ein Rotationskörper ist.

5. Rührvorrichtung (14) nach Anspruch 4, **dadurch gekennzeichnet, dass** der Auftriebskörper (25) eine konische Form aufweist, wobei ein größerer Durchmesser zum Montageende (18) der Rührwelle (17) zeigt.

6. Rührvorrichtung (14) nach Anspruch 4, **dadurch gekennzeichnet, dass** der Auftriebskörper (25) eine scheibenförmige Form aufweist.

7. Rührvorrichtung (14) nach einem der Ansprüche 4 bis 6, **dadurch gekennzeichnet, dass** der Auftriebskörper (25) über ein Drucklager mit dem zweiten Paddel (16) verbunden ist.

## Revendications

1. Dispositif d'agitation (14) pour l'agitation d'un fluide biphasique (5), avec un arbre d'agitation (17) pouvant être monté, par une extrémité de montage (18), dans un entraînement d'agitation, avec une première pale (15) disposée de manière fixe en rotation à ou à proximité de l'extrémité d'agitation (19) de l'arbre d'agitation (17) située à l'opposé de l'extrémité de montage (18), et at avec une deuxième pale (16) disposée de manière fixe en rotation sur l'arbre d'agitation (17) entre l'extrémité de montage (18) et la première pale (15), la deuxième pale (16) étant montée par rapport à l'arbre d'agitation (17) de telle sorte qu'elle puisse être déplacée dans une direction axiale le long de l'arbre d'agitation (17), ce qui permet de modifier la distance axiale entre la deuxième pale (16) et l'extrémité de montage (18) de l'arbre d'agitation (17), **caractérisé en ce que** le dispositif d'agitation (14) comprend un arbre adaptateur (29) qui entoure l'arbre d'agitation (17) et qui peut être monté de manière fixe en rotation au-dessus de la première pale (15), une surface extérieure (30) de l'arbre adaptateur (29) présentant une forme non symétrique en rotation qui permet de monter la deuxième pale (16) par rapport à l'arbre adaptateur (29) de telle manière qu'elle puisse être déplacée dans une direction axiale le long de l'arbre adaptateur (29) et donc dans une direction axiale le long de l'arbre d'agitation (17) qui est entouré par l'arbre adaptateur (29).

2. Dispositif d'agitation (14) selon la revendication 1, **caractérisé en ce que** la première pale (15) est reliée rigidement à l'extrémité d'agitation (19) de l'arbre d'agitation (17).

3. Dispositif d'agitation (14) selon l'une des revendications précédentes, **caractérisé en ce que** la deuxième pale (16) est reliée à un corps de flottaison (25) qui flotte sur une surface supérieure du fluide biphasique (5), ce qui entraîne une montée de la deuxième pale (16) à mesure que le corps de flottaison (25) s'élève avec une surface supérieure ascendante du fluide biphasique (5).

4. Dispositif d'agitation (14) selon la revendication 3, **caractérisé en ce que** le corps de flottaison (25) est un corps rotatif.

5. Dispositif d'agitation (14) selon la revendication 4, **caractérisé en ce que** le corps de flottaison (25) a une forme conique avec un diamètre plus grand faisant face à l'extrémité de montage (18) de l'arbre d'agitation (17).

6. Dispositif d'agitation (14) selon la revendication 4, **caractérisé en ce que** le corps de flottaison (25) a une forme discoïdale.

7. Dispositif d'agitation (14) selon les revendications 4 à 6, **caractérisé en ce que** le corps de flottaison (25) est relié à la deuxième pale (16) par l'intermédiaire d'un palier de butée.
